# EUROPEAN PATENT APPLICATION

(11) **EP 4 506 728 A1**
(43) Date of publication of application: **12.02.2025**
(21) Application number: 23190330.3
(22) Date of filing: 08.08.2023
(51) Int. Cl.: G01S 15/89, A61B 5/00, A61B 8/00, G01H 9/00

(54) **ULTRASOUND IMAGER, TRANSDUCER, AND USE**

(71) Applicant: Nederlandse Organisatie voor toegepast-natuurwetenschappelijk Onderzoek TNO, 2595 DA 's-Gravenhage (NL)
(72) Inventor: HARMSMA, Peter Johan, 2595 DA 's-Gravenhage (NL); QUESSON, Benoit André Jacques, 2595 DA 's-Gravenhage (NL); VAN NEER, Paul Louis Maria Joseph, 2595 DA 's-Gravenhage (NL); VAN DER HEIDEN, Maurits Sebastiaan, 2595 DA 's-Gravenhage (NL); ALTMANN, Robert Karl, 2595 DA 's-Gravenhage (NL); PIRAS, Daniele, 2595 DA 's-Gravenhage (NL)
(74) Representative: V.O.

(57) **Abstract**

The present disclosure provides an imaging tool an opto-acoustical imaging tool, an integrated photonic ultrasound transducer (IPUT), and the use thereof the imaging of (biological) samples. The tool comprises an ultrasound emitter (10) to provide an acoustic probing wave (P) for imaging a sample (100), and an IPUT (20) comprising an optical waveguide (WG) (21) supported by a carrier (22), wherein, the WG (21) and the carrier are essentially non-resonant over a range covering a frequency of the probing wave. The IPUT (20) comprises an amplifier element (30) that is mechanically coupled to at least a part of the WG and having an exposed impingement surface (31) to receive a portion of the acoustic pressure wave, said impingement surface extends along a plane comparatively more distant from the carrier substrate than the WG, and having a plan area greater than the WG.

## Description

### TECHNICAL FIELD AND BACKGROUND

The present disclosure relates to the field of ultrasound imaging. The present disclosure concerns an imaging tool, an integrated photonic ultrasound transducer (IPUT), and the use of the tool and/or IPUT for the opto-acoustical imaging of samples, particularly biological tissue such as human tissue.

The basic principle is that one or more acoustic source sends out an ultrasound signal into a sample. Due to interaction of the ultrasonic wave and the sample, a reflected signal is created. Ultrasound receivers are used to detect an output signal in the form of amplitude and/or phase. Usually a linear/curved/phased array configuration is used to construct a 2D or 3D image. Tomography allows the construction of a three-dimensional image of the tissue. Typically, multiple emitters and/or receivers are used, together with advanced signal optimization and processing for optimum results.

Opto-acoustic receivers comprising a mechanical resonator supporting an optical wave guide for converting an acoustic pressure wave into a modulation of a property of light are known. The mechanical resonance frequency of the receiver is typically matched to the frequency of the ultrasound signal, so that mechanical amplification of the modulation can be obtained.

Typically, sensitive optical sensing of the modulation is performed with Mach Zehnder Interferometers (MZI), Ring Resonators (RR) or similar techniques. The minimal achievable noise equivalent pressure (NEP) of Mach Zehnder Interferometer based Integrated Photonics Ultrasound Transducers (IPUTs) is limited by the optical waveguide properties, e.g. due to foundry specific restrictions, and material platform limitations regarding optical wave guide geometries (minimal bend radius, optical wave guide losses). This limits the amount of optical waveguide loops which fit on a particular membrane.

The minimal bend radius of the optical wave guides also limits the minimal membrane diameter and therefore upper resonance frequency.

EP3851815A1 discloses a ring resonator and a MZI interferometer comprising a sensing waveguide that extends over multiple membranes.

WO2021145766A1, WO2021145768A1 are further examples of disclosures describing a waveguide supported by one or more resonator membrane.

While known devices can provide some benefits, there remains a desire for sensors having increased sensitivity, reduced dimension and/or for operating at comparatively higher acoustic frequencies.

### SUMMARY

The present disclosure aims to mitigate or eliminate one or more of the disadvantages associated with conventional imaging tools. As detailed herein below the present disclosure can provides an on chip integrated IPUT benefiting from improved sensitivity, improved capability of operating at comparatively higher ultrasound frequency, and/or comparatively reduced form factor, while remaining compatible with known microfabrication technology.

Aspects of the present disclosure relate to an imaging tool. The tool comprises at least: i) an ultrasound emitter which is configured to provide an acoustic probing wave that can be used for imaging a sample, and ii) an integrated photonic ultrasound transducer (also referred to as IPUT). The IPUT comprises an optical waveguide (also referred to as WG) that is supported by carrier substrate. In preferred variations, the WG and the carrier substrate are essentially non-resonant over the range of frequencies of the acoustic probing wave. Typically a primary resonance mode of the WG as present on the carrier is at a frequency higher than an operating frequency of the acoustic pressure wave, preferably by a factor of at least ten. Operating the WG in non-resonant mode, e.g. below resonance, advantageously enables a comparatively large bandwidth in combination with a small form factor. To improve sensitivity the IPUT (20) is provided with an amplifier element. The element is an additional structural feature, e.g. a solid layer, fin or pillar, which is mechanically coupled to at least a part of the waveguide. The amplifier element has an exposed impingement surface for receiving at least a portion of an acoustic pressure wave, e.g. as received from a medium of sample to be imaged. The impingement surface extends along a plane that is comparatively more distant from the carrier substrate than the WG. In addition, the impingement surface has an area (in plan view) that is greater than an area of the WG (as viewed from the same direction) the amplifier element is coupled to. Inventors find that realized improvement in sensitivity is at least in part realized by stress and/or displacement as transduced onto the WG as enabled by the comparatively increased surface area for receiving acoustic pressure waves. The stress and/or deflection thusly impacted on the WG can be detected by a modulation of a property of light transmitted by the WG.

The transducer can advantageously be used in combination with one or more dedicated ultrasound generator, e.g. in a pulse-echo mode, e.g. for imaging. Alternatively, or in addition, the transduced can be used as listening device (microphone) to pick up sound generated elsewhere.

In a preferred embodiment, the amplifier element can be understood as comprising a cover layer of a solid composition that is disposed along a face of the WG, e.g. a top face of the WG opposite an interface with the carrier substrate. To provide an extended surface the solid protrudes laterally beyond the WG. In some variations the solid cover layer extends only beyond at least one edge, e.g. side, of at least a section of the WG. In other preferred embodiments the solid cover layer protrudes beyond both sides of the waveguide or waveguide section. For example, in some embodiments the amplifier element may form a cantilever structure that extends on one or two opposing ends across an edge of the waveguide. The longer the extension the higher the additional impingement surface provided can be. In some embodiments, .e.g. in a ring resonator, the solid cover layer covers and/or extends beyond the entire length of WG. Alternatively, or in addition, the waveguide can continue to the input-output ports of the IPUT.

The solid cover layer or at least the impingement surface provided by the amplifier element typically extends in a general direction that is about parallel (within ±5 degrees) to the WG. In this way the tools can be most sensitive to pressure waves impacting about normal to the carrier substrate. To provide directionality the solid cover layer can be oriented under a non-parallel direction relative to the carrier substrate, e.g. in a range > 5° up to 90° (upright) relative to the carrier substrate. Alternatively, or in addition, the cover layer can be tapered (tapering thickness). Further, in some embodiments, the cover layer can be curved or comprise one or more curved, sloped, and/or tapered section.

In some embodiments, the solid cover layer is in a direct mechanical contact with a top face of the WG, e.g. directly disposed or formed onto the WG. Optionally one or more intermediate structures may be provided, including but not limited to buffer layers and pillars.

In a preferred embodiment, the amplifier element extends over a gap separating two adjacent portions of the WG. For example, the amplifier element can cover adjacent portions of a single WG structure, e.g. a loop, spiral, or otherwise adjacent sections of a WG. Alternatively, or in addition, the amplifier element can extend across a gap between adjacent portions of two or more separate waveguides, e.g. a set of parallel waveguides. Bridging a separation between adjacent waveguide portions maximizes an effective impingement areas of the WG structure.

In another or further preferred embodiment, the amplifier element includes a pedestal. Said pedestal can be a free standing pillar or fin, extending from a base at the carrier substrate or the waveguide, in an upstanding direction away from the substrate. The pedestal is typically positioned in a direct vicinity of the WG. The pedestal can be positioned onto the carrier substrate. Alternatively, or in addition, the pedestal can be (partly) provided on top of the WG. Exposed top and/or side faces of the pedestal can provide an impingement face for the acoustic probing wave. Stress/deflections imparted on the pedestal can be effectively transferred to the WG either directly or via the carrier substrate. Accordingly, the sensitivity of the IPUT can be improved by transfer of stress and/or displacement. Similar as for the solid cover layer a shape, dimensioning, and or orientation (e.g. height, length, thickness) of the pedestal can depend on an intended gain as determined by the added impingement area provided. An optimal separation distance between WG and pedestal (when supported by the carrier) can be determined experimentally. Similar as for the solid cover layer the pedestal can be tilted with an angle to add directionality to the sensitivity, e.g. 80-100° (relative to the carrier substrate). Making the pedestal tilted/oriented improves sensitivity in a privileged direction, which is beneficial in some applications, e.g. in industrial applications (e.g. distance sensors). In order to improve the overall directionality (omnidirectionality), as for medical imagers, multiple or sectioned amplifiers (e.g. pedestals) are envisioned.

In some preferred embodiments, a solid top layer is disposed along a top face of the pedestal. The solid top layer can protrude laterally beyond one or more edge of the WG to provide comparative increased impingement surface area. Functionally the top layer can be understood as equivalent to the solid cover layer except being supported by the pedestal and thus more raised from the top faces of the WG.

In some preferred embodiments the amplifier element is also non resonant.

In other embodiments, the amplifier element may be a resonant element, i.e. a mechanical resonator, configured to amplify stresses and/or strains (rather than mere forces) imparted to the WG. For example, the amplifier element can have a primary resonance mode at a frequency of the acoustic probing wave (P). For example, the solid cover layer and/or the solid top layer can have a dimension of (N+0/5) λ, whereby n represents an integer ≥ 0, and λ represents a wavelength of the acoustic pressure wave as received at the IPUT and under account of the sampling medium (e.g. air, water, etc.)

In a particularly preferred variation the amplifier element can be thinned down towards an outer perimeter. Thinning down the amplifier element can be used to reduce an overall weight, while leaving the design (e.g. radius of curvature, form factor, etc.) of the WG unaffected. For example, in a variation portions of the solid cover layer and/or the solid top layer that protrude beyond the waveguide has a decreasing thickness.

To enhance performance the WG can be provided with a plurality of amplifier elements. To provide directionality subsets of the plurality may mutually differ, e.g. differently oriented, shaped, and/or oriented, shaped, etc.

To enable three dimensional ultrasonic imaging the imaging tool can be provided with a plurality IPUTs and/or ultrasound emitters.

As a further option the WG can be provided as a multilayer structure which layers are oriented upright, e.g. normal with respect to the carrier substrate. Orienting the WG in an upright position in combination with provision of the amplifier element improves directional sensitivity of the IPUT, e.g. to acoustic probing waves impinging onto the IPUT in a direction of 45° relative to the carrier.

It will be appreciated that the disclosure is not limited to a specific IPUT or operating principle. Inventors found that the benefits of providing a combination of non-resonant carrier, WG, and/or amplifiers can be applied to so-called interference based IPUTs, whereby the output scales with optical interference between a light pulse from the WG and a reference pulse. In a preferred embodiment, the IPUT comprises a ring resonator type transducer. In another or further embodiment, the IPUT is based on a Mach Zehnder type transducer. It will be understood that in case the WG is a ring shaped element the amplifier element can be disc-shaped. For example, a disc shaped solid layer covering the entire WG bridging the central gap. It will be understood that the disc does not need to hermetically seal the underlying gap. Through holes (e.g. as a result from manufacturing) can be tolerated.

Aspects of the present disclosure further relates to an integrated photonic ultrasound transducer (IPUT) as disclosed herein. For example an integrated chip for use in the assembly of an ultrasound imager. The IPUT can advantageously be configured to operate with comparatively high ultrasound frequency for example, >_ 10 MHz, ≥ 100MHz, or higher, such as ≥ 1GHz or ≥ 10 GHz, up to tens or even one hundred GHz or more, even with the WG being essentially non-resonant over said range. Of course an upper limit can be dependent on carrier and/or WG material and/or dimensioning.

Aspects of the present disclosure further relate to a use of the imaging and/or the IPUT in a method of the imaging of a sample. The sample can be biological tissue, e.g. mammalian or human tissue.

### BRIEF DESCRIPTION OF DRAWINGS

These and other features, aspects, and advantages of the apparatus, systems and methods of the present disclosure will become better understood from the following description, appended claims, and accompanying drawing wherein:
FIGs 1, and 2 detail aspects of known ultrasound imaging tools;
FIGs 3A, 3B, and 3C detail aspects of ultrasound imaging tools in accordance with the present disclosure;
FIGs 4A, 4B, and 4C detail aspects of embodiments of ultrasound imaging tools in accordance with the present disclosure;
FIG 5 detail aspects of embodiments of ultrasound imaging tools in accordance with the present disclosure;
FIGs 6A and 6B, 7A and 7B provide FEM simulated results;
FIG 8 detail aspects of embodiments of ultra sound imaging tools in accordance with the present disclosure;
FIGs 9A and 9B provide FEM simulated results;
FIGs 10A and 10B detail aspects of embodiments of ultrasound imaging tools in accordance with the present disclosure with an overlay of FEM simulated data;
FIGs 11A and 11B provide FEM simulated results for the variation shown in FIGs 10A-B;
FIGs 12A and 12B detail aspects of embodiments of ultrasound imaging tools in accordance with the present disclosure;
FIGs 13A to 13G provide FEM simulated results for the variation shown in FIGs 12A;
FIG 13H to 13O provide further subpictures;
FIGs 14A, 14B, and 14C detail aspects of embodiments of ultrasound imaging tools in accordance with the present disclosure; and
FIGs 15, 16 and 17 schematically illustrate aspects of manufacturing methods.

### DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

Conventional IPUTs, or on-chip photo-acoustic receivers, typically comprise a membrane that is configured to deflect (optimally at resonance) in response to an acoustic probing signal. Deflection is higher at resonance frequencies, therefore the resonance frequency is adjusted to the expected acoustical resonance frequency. However, the IPUT works at static pressure as well.

An optical waveguide structure that is at least in part disposed onto the membrane is used as a sensing element. The WG can comprise a ring resonator or interferometer based configuration and acts as a wavelength specific filter (in amplitude and/or phase). The membrane deflection alters the wavelength response, which can be accurately detected, e.g. as a modulation of a property of light. A common way to read-out a ring resonator at high speed comprises setting a laser line to the flank of a resonance (e.g. where the transmission is appr. 0.50). Shifts in wavelength resonance due to the deflection caused by the acoustic signal can thus be followed as changes in the observed transmission through the ring resonator. Alternative methods exist, e.g. using two ring resonators in series, or by using an interferometer such as a Mach Zehnder interferometer configuration (MZI) or other phase sensitive optical read-out principles.

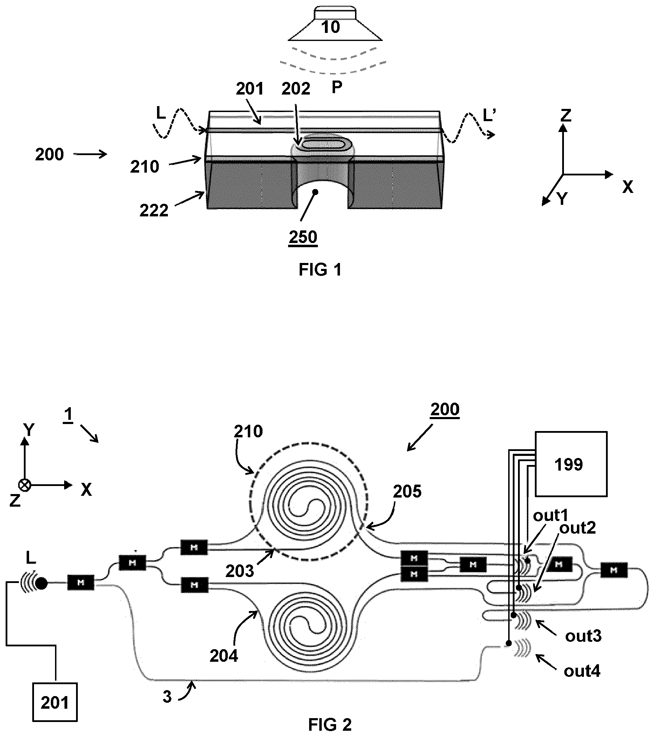

FIG 1 illustrates aspects of an IPUT 200 based on an on-chip ring resonator (redrawn from a publication by S.M. Leinders, et al in Nature Scientific Reports, 14328, 2015). As illustrated the device 200 comprises a carrier substrate 222. A membrane 210 extends over an aperture (or recess) 250 in the substrate. The membrane deflects in response to an acoustic pressure wave (P). The mechanical resonance frequency of the membrane is matched to the frequency of the ultrasound signal as emitted by emitter 10.

A dedicated ultrasound emitter is not essential for operation. The IPUT can also be used for passive applications, e.g. listening in/detection of environmental noise or laser induced ultrasound.

A WG structure is provided which includes a ring resonator WG 202 and an auxiliary WG 201. The ring resonator deflects/deforms with deflections of the membrane in response to an incoming pressure wave P from ultrasound emitter 10. Auxiliary WG 201 that is optically coupled to the ring is used to probe a modulation of light (L, L'). Note that, for ease of understanding, the illustration does not depict components including but not limited to light source(s), splitters, optical power reference elements, and read out electronic.

FIG 2 illustrates an ultrasound imaging tool 1 based on interferometric read-out. Similar to imagers according to the present disclosure, the imager can be an assembly comprising an IPUT 200, an ultrasound emitter 10 (not shown), a light source 201, and readout electronics 199. As mentioned before a dedicated ultrasound emitter is not essential. Note that, as for imagers according to the present disclosure, one or more components of the and/or the readout electronic source may be integrated in part of full in the IPUT.

The WG structure is a MZI-based interferometer configuration. The long spirals 203 and its unlabeled equivalent in the lower branch form one high-sensitive interferometer. The short loops 205 and 204 form a low-sensitive interferometer. The optical power reference is labeled 3 and routes to out 4. As for the configuration described in relation to FIG 2, WG 203 extends along resonator membrane 210 configured to resonate at a frequency of a received probing wave (P). The boundary of the membrane is indicated by a dashed line. To increase a sensitivity WG 203 follows a meandering trajectory (spiraling) over the membrane. Alternatively, the interferometer may be of the 3x3 type comprising couplers with 3 outputs to obtain signals with a mutual phase difference of 120°.

The lowest pressure (Noise Equivalent Pressure) that a sensor according to the known configurations can detect depends, for a given waveguide cross sectional geometry, mainly on the membrane diameter, it's elastic properties, the position of the WG on the membrane, the total length of the optical waveguide, and losses in said waveguide. Maximum length of a trajectory, e.g. number of spiral loops, on a membrane is determined by the membrane radius and a minimum allowable bend radius of optical waveguide. The membrane resonance frequency (fᵣₑₛ) scales inversely with its area (e.g. 1/diameter(squared) for a circular membrane. This implies an upper operating frequency limit. Therefore the achievable Noise Equivalent Pressure is limited by the geometrical limits regarding the optical wave guides and membranes and the optical wave guide losses, even when combining multiple interferometers or ring resonators in series. As such, the minimal achievable noise equivalent pressure (NEP) of known devices, e.g. Mach Zehnder Interferometer/Ring Resonator based IPUTs, is limited by foundry and material platform limitations regarding optical wave guide geometries (minimal bend radius, optical wave guide losses).

Thus a practical maximum mechanical resonance frequency limitation for IPUTs exists. Room to reduce a WG bending radius are restricted because optical losses will progressively increase as the waveguide radius becomes smaller. Options to use smaller membranes (having a higher resonance frequency fᵣₑₛ) are limited due to a minimum bending radius of the waveguides. Options to use thicker membranes (having a higher resonance frequency fᵣₑₛ) are limited due to reduced membrane deflection with increasing thickness, and fabrication constraints.

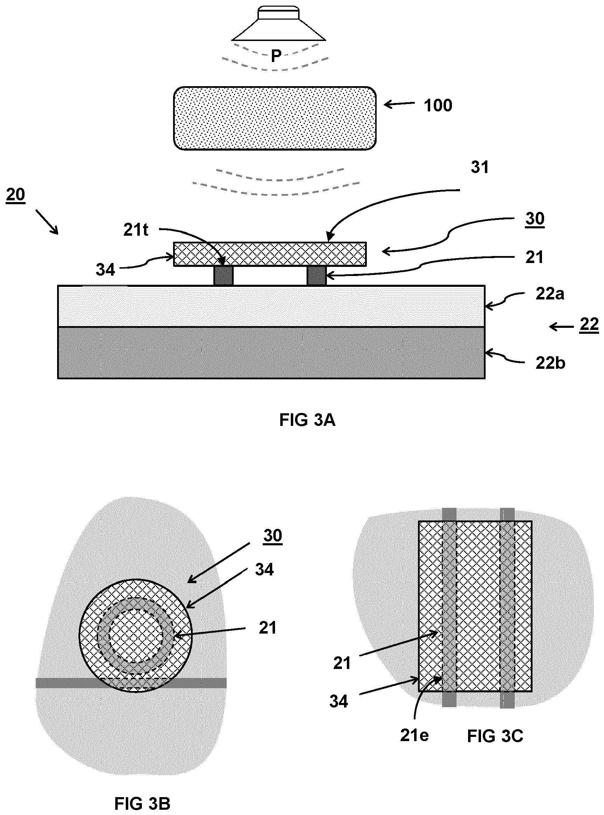

The present disclosure as explained in further detail with reference to FIGs 3-17 mitigates or eliminates the limitations associated to the known IPUTs.

FIGs 3A schematically illustrates an imaging tool 1 comprising:
i) an ultrasound emitter 10 that is configured to provide an acoustic probing wave P for imaging a sample 100, and
ii) an integrated photonic ultrasound transducer IPUT 20 comprising an optical waveguide WG 21 supported along a face 21t of a carrier substrate 22, wherein,
the WG 21 and the carrier substrate are essentially non-resonant over the range of frequencies of the acoustic probing wave. The IPUT 20 comprises an amplifier element 30 that is mechanically coupled to at least a part of the waveguide, said amplifier element having an exposed impingement surface 31 to receive a portion of the acoustic pressure wave from the sample, said impingement surface extending along a plane comparatively more distant from the carrier substrate than the WG, and having a plan view area greater than a plan view area of the WG coupled thereto. The IPUT structures according to the present disclosure benefit from mechanical amplifiers, that enhance the strain and/or the stress experienced by the waveguide. As explained in further detail below the amplifier can optionally in some variations be resonance matched with the acoustic probing wave (P) to amplify stress/strain transduction to the WG.

Note that the substrate 22 may be a multilayer substrate comprising sublayers 22a, 22b (FIG 3A). As seen in FIG 1 and 3A the impingement surface 31 acts as a receiver at which acoustic probing waves (P) as received from a sample 100 are collected. Of course the probing wave may be from other sources including but not limited to environmental noise and laser induced pressure waves. As illustrated the impingement surface 31 typically protrudes over one or both lateral edge(s) 21e of the WG. The additional surface and waveguide together can act as a mechanical resonator with a resonance frequency above the operating frequency. The stress-induced refractive index change can cause a phase shift in the waveguide (and thus a resonance shift in the ring resonator or phase shift in the MZI). Advantageously, the additional impingent surface + waveguide can be smaller / stiffer than a membrane of a conventional device, and thus the resonance frequency can be significantly higher.

In some variations, e.g. as illustrated in FIGs 3A-3C, the amplifier element 30 extends over a gap between two separate WGs or adjacent portions of a single WG. FIG 3B (top view) illustrates a variation whereby the amplifier element 30 is a disc shaped solid cover layer 34 which is positioned onto and extends over an outer perimeter of a ring shaped resonator WG 21 (covered by solid cover layer 34 as indicated by dashed lines. FIG 3C (partial top view) shows a variation wherein the solid cover layer 34 is disposed onto sections of a spiral WG 21. Note that the concept is not limited to spiral WGs.

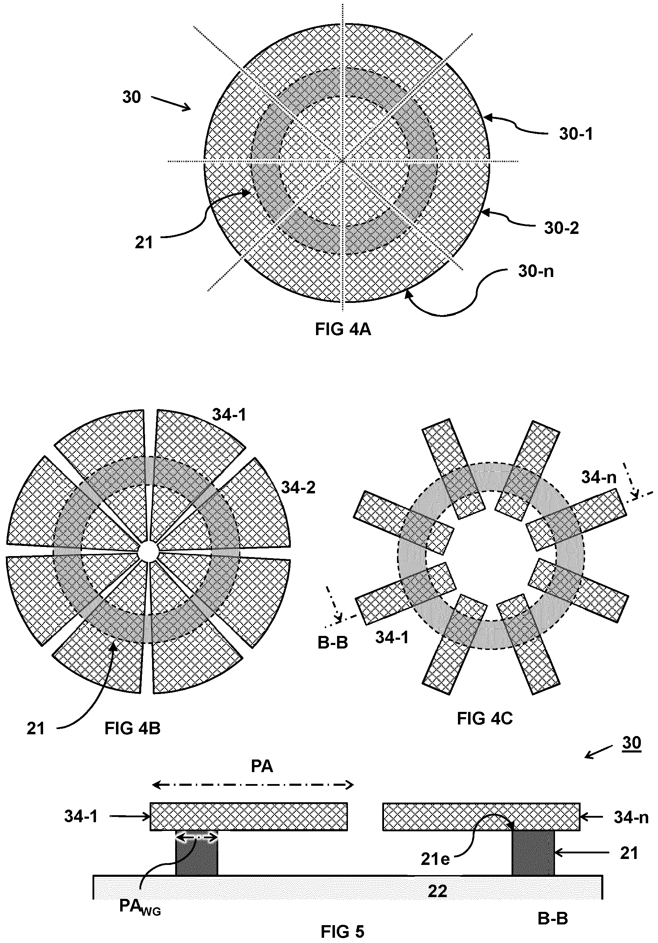

FIGs 4A, 4B, and 4C detail aspects of other or further embodiments of ultrasound imaging tools in accordance with the present disclosure. As best seen in FIG 4B and 4C, the IPUT comprises one or more of the amplifier elements that are mechanically coupled to one or more further parts of the WG 21. Associating the WG with a plurality of amplifier element 30 can increase an overall sensitivity of the IPUT, as compared to variations having a lower coverage.

In some embodiments, e.g. as shown in FIG 4A, the amplifier element 30 can be interpreted conceptually as being divided into a plurality of separate elements 30-1,30-2,30n. The modulation of the light is cumulated by all amplifiers due to the acoustic wave, and each amplifier has its own high resonance frequency, leading to a large effective acoustic bandwidth.

FIGs 4B and 4C illustrate variations comprising an integer number n of solid cover layers (34-1, 34-2, 34-n). FIG 5 provides a cross-section side view of the embodiment shown in FIG 4C along direction B-B.

In a preferred embodiment, the amplifier element 30, e.g. the solid cover layer 34 (as detailed further herein below) can be configured to have stiffness along one of the principle directions within said element to provide directional sensitivity to incoming acoustic probing waves (P). For example, the amplifier element 30 may be cantilever shaped (Fig 4C), wedge shaped (FIG 4B), or otherwise, whereby wedge shaped elements can advantageously provide a larger aerial coverage of the underlying WG 21. See also Fig 5 indicating a plan area PA of the impingement surface realized by the solid cover layer 34 as compared to the PA_{WG} of the covered WG portion. The isotropic elements, e.g. cantilevers, or alternatives with a well-chosen geometry, can resonate in 1 direction at significantly lower frequencies than in the others (albeit still significantly larger than for conventional membrane configurations at least in part due to its relatively small size compared to the membrane. This implies that, even when operating outside a resonance window (e.g. below a primary resonance mode) of the element, that the response along said direction will be significantly larger than the response in direction orthogonal thereto. Combined with a realization that the amplitude and Q-factor is higher for cantilevers as compared to membranes it follows that a significant benefit in response can be realized at small sizes and thus high frequencies.

Of course it will be appreciated that the disclosure is not to be interpreted as construed to the illustrated embodiments. For example, different shaped WGs and/or solid cover layer 34 are envisioned. For a ring resonator, a disk can be a logical shape.

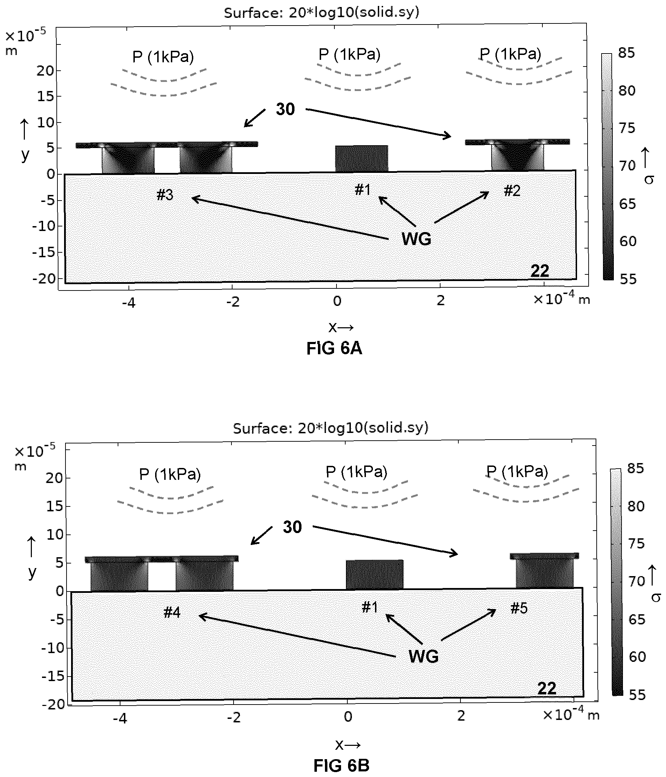

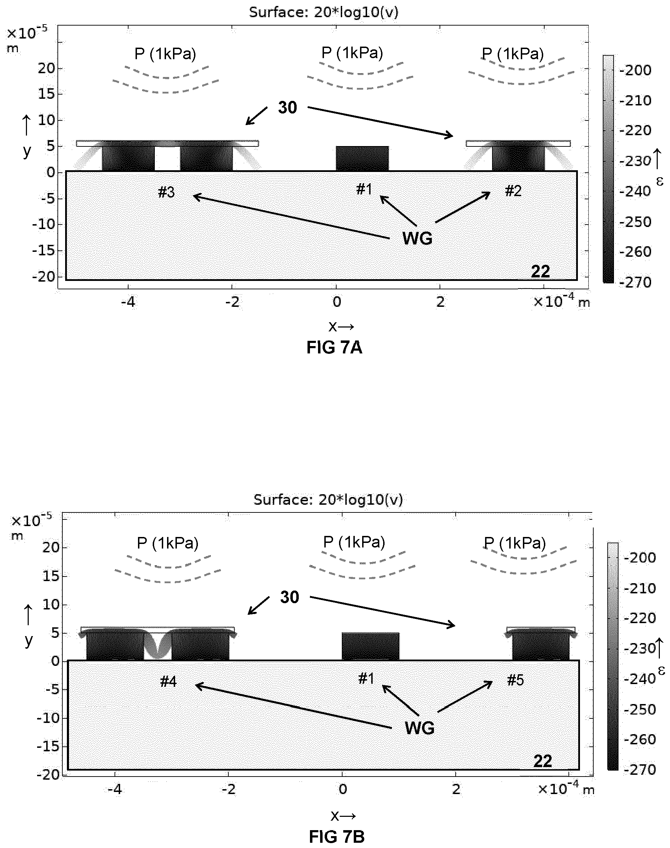

FIGs 6A and 6B, 7A and 7B provide 2D-FEM simulated results comparing a response of conventional bare WGs and exemplary WGs comprising an amplifier element 30 as disclosed herein in terms of stress (σ) and strain (ε) to a static pressure pulse at 1 kPa. The WGs all have a lateral dimension (x) of 100 µm and a thickness of 50 µm. FIG 6A illustrates a stress (σ) response of a bare WG (marked #1) to WGs (#2, #3) with a solid cover layer. In sample #2 the solid cover layer extends over a single WG. In sample #3 the WG spans over to adjacent WG portions. In either case the cover protrudes laterally by 50 µm. FIG 7A depicts the corresponding response in terms of strain (ε). FIGs 6B and 7B differing in that the cover layer in samples #4 and #5 protrudes significantly less. Both strain and stress are given in dB. For example, the stress [55 - 85] dB can be read in linear values in N/m² as follows 20*log10(x)=55dB → ~560N/m². Similarly the displacement (and hence stress) [-200 -270] dB can be read in meters. As shown, a provision of the cover layer has a pronounced effect on the stress/strain experienced by the WG. Embodiments with longer cantilevers (#2 and #3) improve the strain experienced in the waveguide compared to the bare waveguide and shorter cantilevers (#4 and #5). With little difference between corresponding bridging and non-bridging variations. In terms of experience stress a clear relation was found in the transduced response (#2 and #3 > #4 and #5 > bare WG), with bridged configurations more suitable for stress sensing.

FIGs 6A and 6B, 7A and 7B provide FEM simulated results;

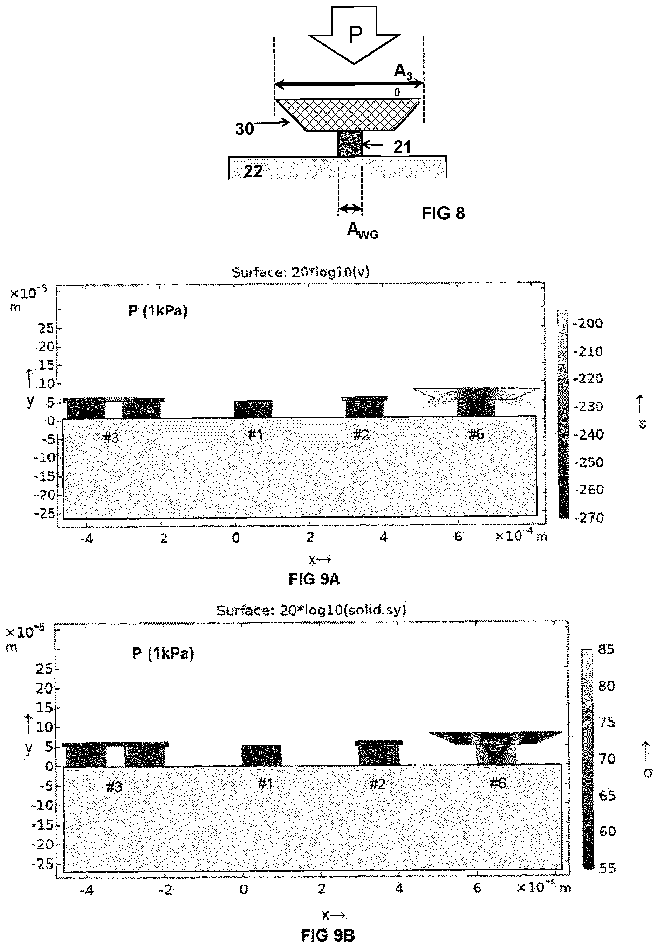

FIGs 8, 9A and 9B detail aspects of embodiments of ultrasound imaging tools in accordance with the present disclosure, whereby portions of the solid cover layer and/or the solid top layer that protrude beyond the waveguide have a decreasing thickness. The embodiments as shown can be regarded as a variation of the embodiment described in FIG 7A (sample #2) and FIG 7B (#5). Specifically: #1 in FIG 9A-B correspond to #1 in Figs 6A-B and 7A-B; #2 in FIG 9A-B correspond to #5 in Figs 6B-6B; 3# in FIG 9A-B correspond to #4 in Figs 6B-7B. In the embodiment the bare WG itself is already partly sensitive to an incoming pressure field. By adding a small mechanical amplifier (like a mechanical horn) on top, the stress can be enhanced. Also the structure can be more robust. The embodiment as shown differs in that the cover layer has a thickness that tapers towards a minimum at its protruding ends. Accordingly, a configuration is formed having a 'mushroom shaped' cross section. The mushroom allows for a large deformation of the disk. Such deformations induce a concentrated stress at the foot of the mushroom, the foot being directly the waveguide. The gain factor can be regarded as the ratio of the surface on top (A₃) vs surface of the waveguide (A_{WG}). As before the mechanical amplifier on top of the waveguide enlarges the sensing area and concentrates the stress at the waveguide.

By reducing a thickness of the cover layer towards its protruding ends the added mass of the element can be minimized, thus enabling a high operating frequency. Similar as described before the cover layer can, be need not, be applied along the entire length of the WG (e.g. a ring resonated covered by a disc).

The amplifier can be used in resonant mode or below-resonance mode.

FIGs 9A and 9B show the response in terms of stress and strain for an exemplary mushroom type configuration to samples marked #1 to #3 as discussed before. As indicated the mushroom shape can be particularly effective in focusing stress and strain onto the WG.

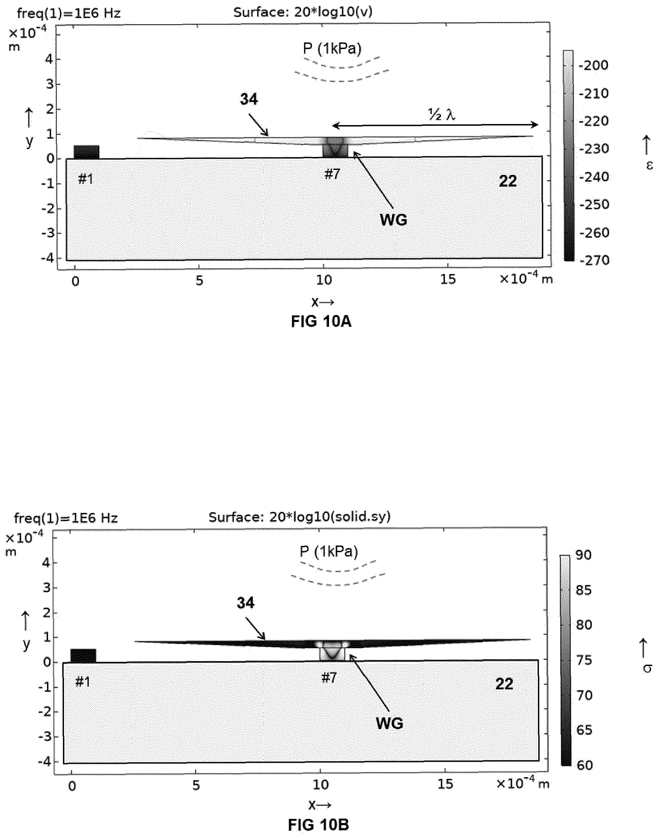

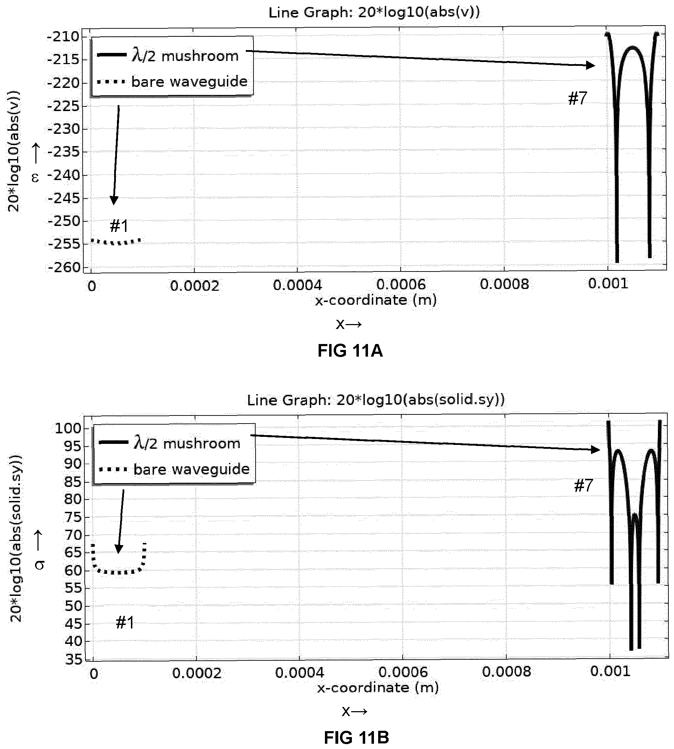

FIG 10A-B and 11A-B illustrate a further variation wherein the amplifier element was effectively enlarged, e.g. as an acoustic wavelength on top, while still having the same footprint of the waveguide. The amplifier can have a resonance mode at a frequency of the acoustic probing wave (P). In the variation as shown there is provided a mushroom shaped configuration wherein the protruding portion has a length of half the wavelength of the incoming pressure filed (1/2 λ). As compared to the bare WG (#1) the corresponding WG (#7) having a resonant solid cover layer 34 was found to provide a 45dB improvement in max displacement (ε) at the WG and a 40dB improvement in the stress profile, proving an ~40dB gain by increasing the sensing area (see also the traces provided in FIGs 11A and 11B)

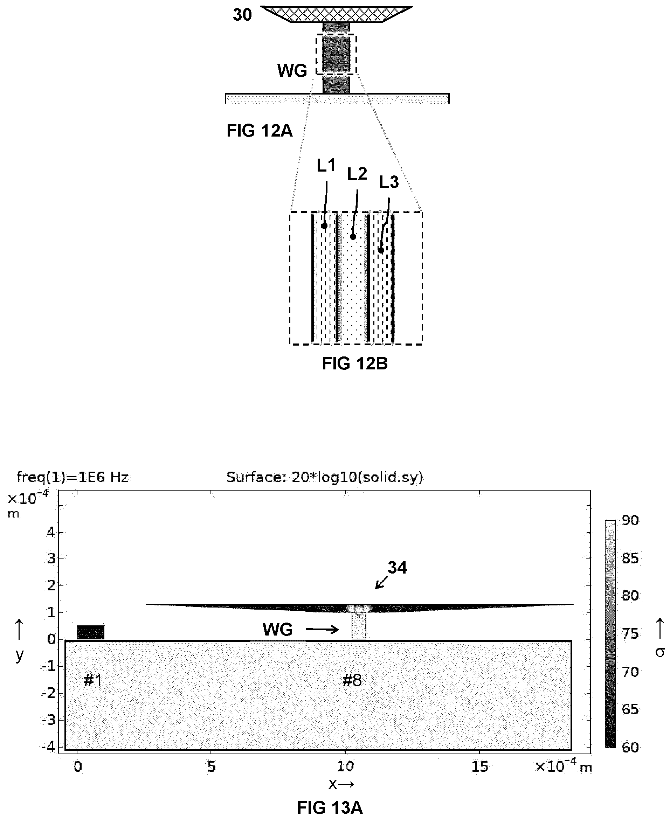

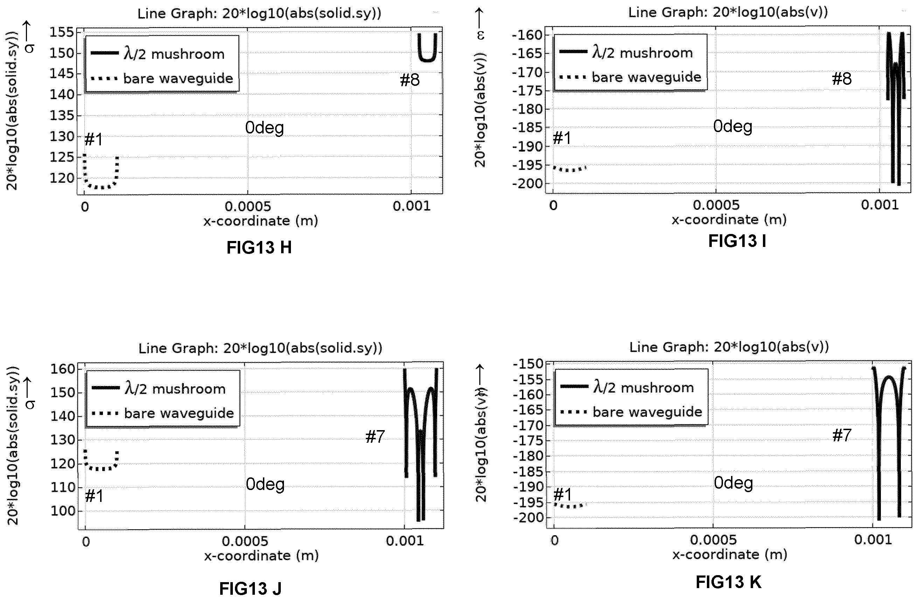

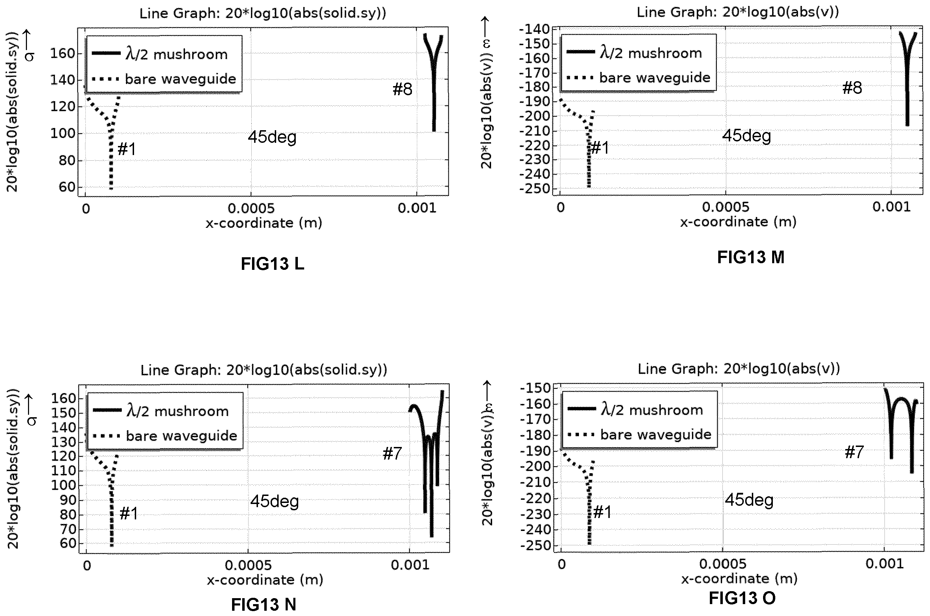

FIGs 12A, 12B, and 13A to 13G detail aspects of embodiments of ultrasound imaging tools in accordance with the present disclosure, wherein the WG is oriented sideways onto the carrier (on edge as compared to Figs 1-11). Note that WGs normally have a width>height. FIG 12A and 12B illustrate a variation wherein the WG is a multilayer structure in which layers (cladding L1, optically transparent core L2, and cladding L3) are oriented upright (about 90±5°) with respect to the carrier substrate, e.g. normal thereto.

It will be appreciated that while the embodiment as shown comprises a tapered solid cover layer the concept can be applied to other configurations as disclosed herein including but not limited to plane cover layers and/or cover layers that bridge adjacent WGs or WG portions, and with resonant and non-resonant amplifier elements.

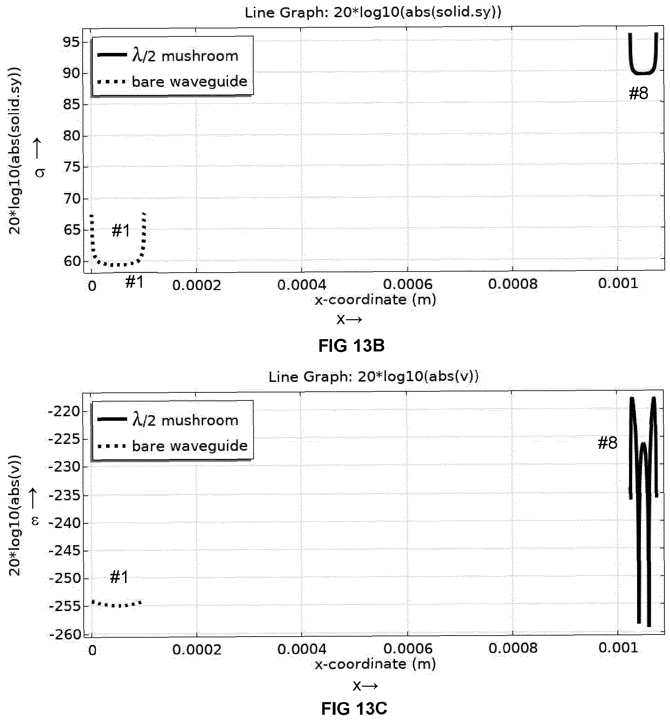

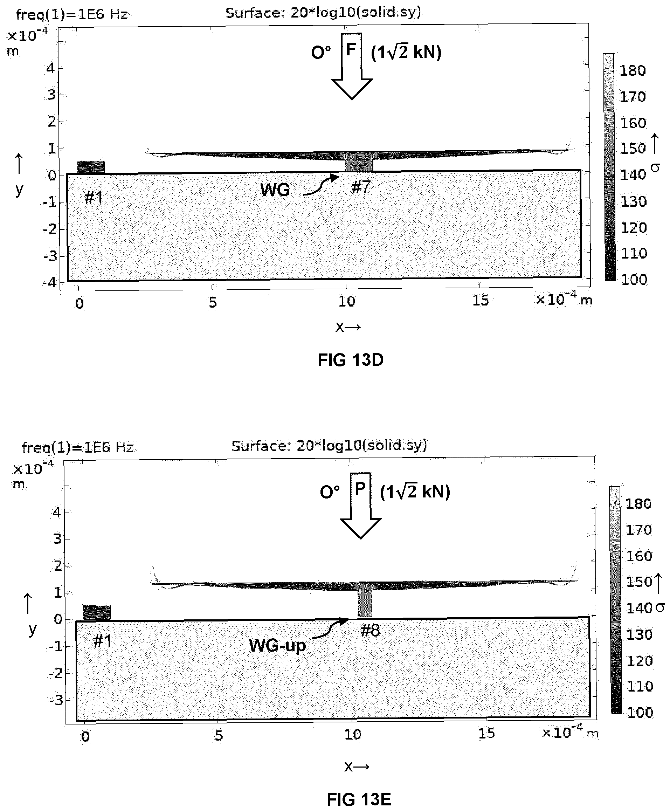

FIGS 13A-C provide FEM simulated results comparing a response of conventional bare WGs (#1) and exemplary WGs (#8) comprising a flipped (rotated 90°) WG covered by a resonant version of a tapered solid cover layer 34, wherein FIG 13 A illustrates an observed stress (σ) transferred to the WG under exposure to a 1 J2kN total force wave impacting normal onto the solid cover layer 34, FIG 13B and 13C provide the corresponding traces for maximum stress and strain (ε). Inventors found that under normal exposure the gain for this configuration, as compared to the bare WG, is about 30 dB, which is somewhat less than for a normal oriented WG with a comparable amplifier element. See also FIGs 13D and 13E comparing the strain response for configurations with the in plane oriented WG (#7) and the upright WG (#8).

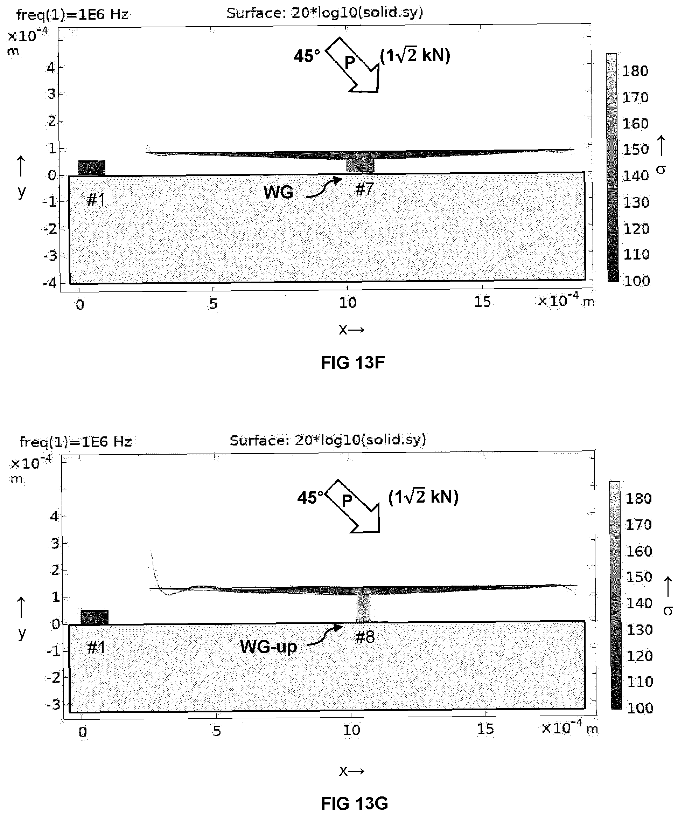

Inventors surprisingly found that the benefit of providing an upright waveguide (WG-UP) strongly increases when the acoustic probing wave (P) impacts the IPUT under a non-right angle. See e.g. FIGs 13F and 13G from which it was be concluded that the gain (under 45° exposure) for version #7 is about 30dB respectively 40dB as compared to bare a WG in terms, whereas the improvement over a bare WG for version with an upright WG (#8) was 45 dB and 50dB respectively. Note that the benefit by changing from an in-plane vs. upright-WG is associated to inverting the compressional stiffness with a bending stiffness of the waveguide.

FIG 13 H to O provide further subpictures, illustrating observed stress and strain for bare and mushroom type WGs. Note that #1, #7, and #8 in Fig 13 H-correspond to variations #1, #7, and #8 in Fig 13 D-G.

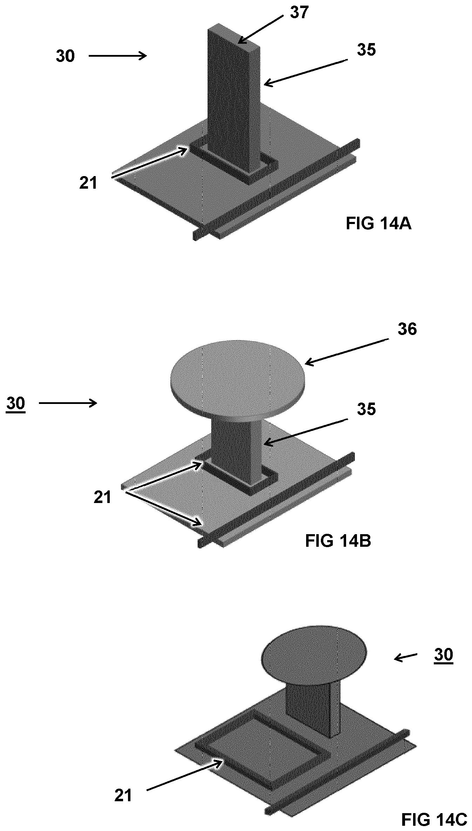

FIGs 14A, 14B, and 13B detail aspects of embodiments of ultrasound imaging tools in accordance with the present disclosure, wherein the amplifier element 30 includes an upstanding pedestal 35 that extends from a base at the face of the carrier substrate 22 beside a section of the WG 21.

FIG 14A illustrates a variety wherein the pedestal is a fin-shaped (elongated) solid element position directly beside a ring resonator WG. Forces imparted on the sides and top of the fin are coupled to the WG via the substrate. Alternatively, the pedestal 35 may be provided directly onto of the WG.

FIG 14B illustrates a variety further comprising a solid top layer 36 disposed along a top face 37 of the pedestal 35. As illustrated the solid top layer 36 can protrude laterally beyond the WG to increase exposed surface area for incoming acoustic probing waves (P). Note that, as compared to the variety shown in FIG 14A, embodiments with a parallel cover layer can be more suited to configurations whereby the acoustic probing wave (P) impinges normal to the carrier, whereas fins can offer increased directional sensitivity. It will be understood that versions with the pedestal may comprise one or more of the following features: converging edge portion (cf FIG 12A) to reduce a weight of the amplifier element; and resonant pedestal and/or a resonant top layer (cf. Fig 10A). Likewise it will be understood that incorporation of a pedestal-based configuration is not to be interpreted as limited to resonator type WGs (e.g. as depicted), but can be equally applied to other WG structures as in MZI type interferometers. For ring resonator type WGs it can be preferred to position the pedestal outside the ring to mitigate counteracting positive and negative contributions to strain/stress.

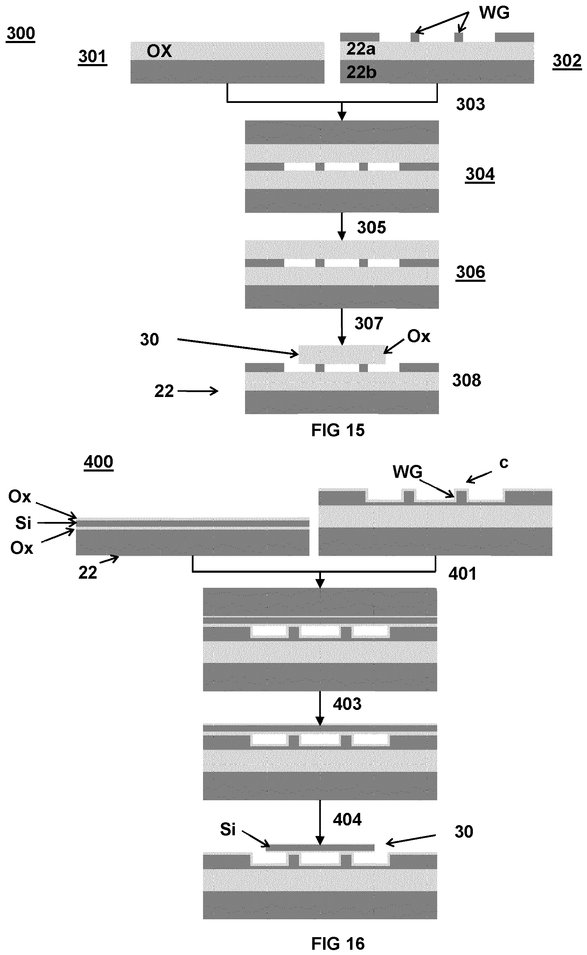

The embodiments as disclosed herein can advantageously be manufactured using common microfabrication methods on commercial platforms. FIGs 15, 16 and 17 schematically illustrate exemplary aspects of manufacturing methods.

FIG. 15 illustrates a method 300 starting from wafer bonding 303 a substrate 301 comprising an oxide top layer onto substrate 302 comprising a WG structure. The WG may be silicon, SiN, polymer-based, etc. or a multilayer stack having a cladded core with an exterior cladding. The resulting stack 304 is etched back in step 305 to the oxide layer (OX), which stack 306 is patterned in 307 (e.g. etched) to define the IPUT (308) amplifier element 30 covering the WG.

In a variation, e.g. as indicated in FIG 16, a method 400 is provided that differs in that a substrate comprising an embedded membrane layer (marked Si) is wafer bonded 401 onto a substrate comprising a WG structure. The membrane can be a Si-membrane. The WG structure may be covered with an optional cover layer (c), e.g. an oxide. The top substrate is etched back 403 to expose the buried membrane, which is patterned 404 to form the amplifier element 30. Using a membrane material that does not have internal stress at operating temperatures such as crystalline silicon, as opposed to common dielectric layers such as oxide or nitride, leads to higher fabrication reproducibility and yield.

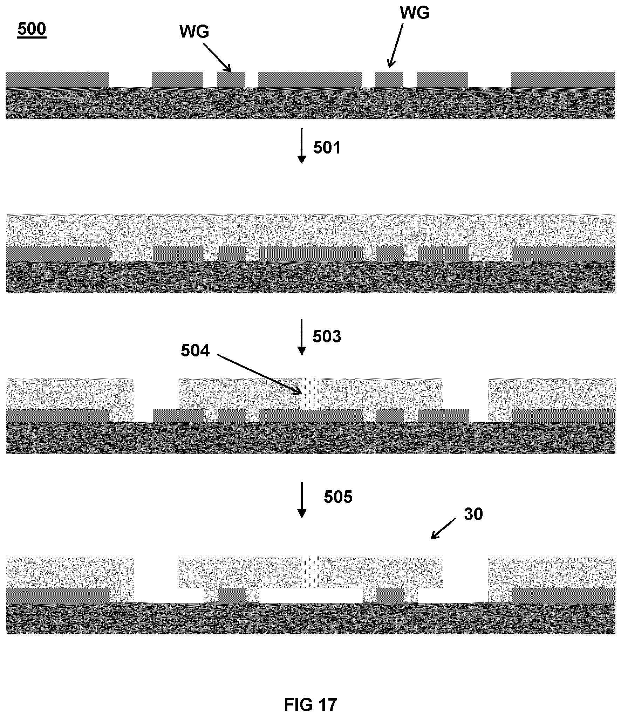

In another or further embodiment, e.g. as illustrated in FIG 17, the method 500 comprises the steps of Oxide deposition 501 and an etch step 503 to define amplifier element 30, followed by an anisotropic etch, whereby etch step 503 includes defining of through holes 504 to facilitate under etching in the subsequent Si etch 505. Note that through holes 504 are dimensioned to be acoustically insignificant.

For the purpose of clarity and a concise description, features are described herein as part of the same or separate embodiments, however, it will be appreciated that the scope of the invention may include embodiments having combinations of all or some of the features described. Of course, it is to be appreciated that any one of the above embodiments or processes may be combined with one or more other embodiments or processes to provide even further improvements in finding and matching designs and advantages.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. Where one claim refers to another claim, this may indicate synergetic advantage achieved by the combination of their respective features. But the mere fact that certain measures are recited in mutually different claims does not indicate that a combination of these measures cannot also be used to advantage. The present embodiments may thus include all working combinations of the claims wherein each claim can in principle refer to any preceding claim unless clearly excluded by context.

## Claims

1. An imaging tool (1) comprising
i) an ultrasound emitter (10) configured to provide an acoustic probing wave (P) for imaging a sample (100), and
ii) an integrated photonic ultrasound transducer (IPUT) (20) comprising an optical waveguide (WG) (21) supported along a face of a carrier substrate (22), wherein,
the WG (21) and the carrier substrate are essentially non-resonant over the range of frequencies of the acoustic probing wave, and wherein
the IPUT (20) comprises an amplifier element (30) that is mechanically coupled to at least a part of the waveguide, said amplifier element having an exposed impingement surface (31) to receive a portion of the acoustic pressure wave from the sample, said impingement surface: extending along a plane comparatively more distant from the carrier substrate than the WG, and having a plan area greater than a plan area of the WG coupled thereto.

2. The imaging tool (1) according to claim 1, wherein the amplifier element (30) comprises a solid cover layer (34) disposed along a top face of the WG and protruding laterally beyond the WG.

3. The imaging tool (1) according to claim 1 or 2, wherein the amplifier element (30) extends over a gap (33) separating two adjacent portions of the WG.

4. The imaging tool (1) according to any of the preceding claims, wherein the amplifier element (30) includes an upstanding pedestal (35) extending from a base at the face of the carrier substrate (22) beside a section of the WG.

5. The imaging tool (1) according to the preceding claim, further comprising a solid top layer (36) disposed along a top face (37) of the pedestal (35)

6. The imaging tool (1) according to any of the preceding claims, wherein the amplifier element has a resonance mode that is excitable by a frequency of the acoustic probing wave (P).

7. The imaging tool (1) according to any of the preceding claims, wherein the portions of the solid cover layer (34) and/or the solid top layer (36) that protrude beyond the waveguide have a decreasing thickness

8. The imaging tool (1) according to any of the preceding claims, wherein the solid cover layer (34) and/or the solid top layer (36) has a preferential direction of vibration.

9. The imaging tool (1) according to any of the preceding claims, comprising one or more further one (30-1,30-2) of the amplifier element (30) that are mechanically coupled to one or more further parts of the WG (21).

10. The imaging tool (1) according to any of the preceding claims, wherein the WG is a multilayer structure, which layers are oriented upright [normal] with respect to the carrier substrate (22).

11. The imaging tool (1) according to any of the preceding claims, wherein the IPUT is a ring resonator type transducer or a Mach Zehnder type transducer.

12. The imaging tool (1) according to any of the preceding claims, wherein the IPUT is a ring resonator type transducer with a ring shaped WG, and wherein the amplifier element (30) comprises a solid cover layer (34) disposed along a top face of the ring shaped WG and bridging the ring shaped WG.

13. The imaging tool (1) according to any of the preceding claims comprising a plurality of the ultrasound emitter (10) and/or the IPUT (20).

14. An integrated photonic ultrasound transducer (IPUT) (20), preferably as specified under ii) according to any of claims 1-13, comprising an optical waveguide (WG) (21) supported along a face of a carrier substrate (22), wherein,
the WG (21) and the carrier substrate are essentially non-resonant over a range of frequencies of an acoustic probing wave, and wherein
the IPUT (20) comprises an amplifier element (30) that is mechanically coupled to at least a part of the waveguide, said amplifier element having an exposed impingement surface (31) to receive a portion of the acoustic pressure wave from the sample, said impingement surface: extending along a plane comparatively more distant from the carrier substrate than the WG, and having a plan area greater than a plan area of the WG coupled thereto.

15. Use of the imaging tool (1) according to any of the preceding claims 1-13 or the IPUT according to the preceding claim for the imaging of a sample, preferably human tissue, using an ultrasound generator operating at an acoustic probing wave having a frequency > 10 MHz.
